# EUROPEAN PATENT APPLICATION

(11) **EP 4 666 974 A1**
(43) Date of publication of application: **24.12.2025**
(21) Application number: 25177815.5
(22) Date of filing: 20.05.2025
(51) Int. Cl.: A61B 18/14, A61B 18/00

(54) **NEEDLE TIP FOR HANDPIECE WITH NFC FUNCTION**

(30) Priority: 11.06.2024 KR 20240075419
(71) Applicant: VIOL Co. Ltd., Bundang-gu Seongnam-si, Gyeonggi-do, 13510 (KR)
(72) Inventor: LEE, Sangjin, 13510 Seongnam-si, Gyeonggi-do (KR); KIM, Jongkil, 13510 Seongnam-si, Gyeonggi-do (KR)
(74) Representative: Zimmermann, Tankred Klaus

(57) **Abstract**

Disclosed is a needle tip structure for a handpiece having an NFC function. The needle tip includes a body mounted at the front end of a handpiece, a first substrate fixed within the body and connected to a plurality of RF supply terminals of the handpiece, a second substrate on which a plurality of micro needles is mounted and movably installed within the body, a holder coupled to a driving shaft of the handpiece through the first substrate and configured to support the second substrate, and a pair of connection pins connected to the second substrate and the first substrate. An NFC chip capable of short-distance wireless communication with an external NFC terminal is installed in the first substrate.

## Description

### [Technical Field]

The present disclosure relates to a needle tip that is used in a handpiece for skin beauty treatment and treatment and, more particularly, to a needle tip for a handpiece having a near field communication (NFC) function capable of performing wireless communication with the outside in a contactless manner.

### [Background Art]

Various procedures for skin beauty treatment or obesity treatment are recently developed. There is a growing interest in a procedure that is performed in an invasive manner, among such various procedures.

A radio frequency (RF) skin beauty treatment (or treatment) device using a needle electrode includes a needle electrode that penetrates a skin tissue, a handpiece to which the needle electrode is coupled, and an energy source that is connected to the handpiece and that transmits RF energy to the needle electrode. The RF skin beauty treatment (or treatment) device operates according to the principle in which the needle electrode penetrates a desired portion within the skin and the RF energy is transmitted to the skin tissue.

In this case, in order to guarantee hygiene, the needle electrode is manufactured in the form of a separate disposable product and detachably mounted on the handpiece. That is, a tip including the needle electrode is manufactured in the form of a consumable part that is detachably mounted at the end of the handpiece and can be replaced and mounted, if necessary.

However, the needle tip for the handpiece, which is manufactured in the form of a consumable part as described above, is manufactured in various types according to the purposes of treatment of the skin. Accordingly, there is a need to efficiently manage the needle tip.

### [Documents of Related Art]

### [Patent Document]

Korean Patent Application Publication No. 2021-0138959 (November 22, 2021)

### [Summary of Invention]

### [Technical Problem]

Embodiments of the present disclosure provide a needle tip for a handpiece having an NFC function, which can be effectively used in unique information, inventory management, and history management of the needle tip because an NFC chip capable of performing wireless communication with the outside in a contactless manner is installed within the needle tip mounted on a handpiece.

### [Solution to Problem]

In an embodiment, a needle tip for a handpiece provided at a front end of the handpiece for skin beauty treatment and treatment in a way to be replaceable and mountable includes a body detachably coupled to the front end of the handpiece, a first substrate fixed and installed within the body, having an opening formed at a center thereof, and electrically connected to a plurality of radio frequency (RF) supply terminals exposed to the front end of the handpiece, a second substrate movably installed within the body and having a plurality of micro needles mounted thereon, a holder having one end coupled to a driving shaft of the handpiece through the opening of the first substrate and having the other end coupled to the second substrate, and a pair of connection pins connected to the second substrate and configured to electrically connect the first substrate and the second substrate. An NFC chip in which information related to the needle tip is stored and configured to perform contactless wireless communication with an external NFC terminal is installed in the first substrate.

In this case, a plurality of RF input electrodes connected to the plurality of RF supply terminals may be formed on a first surface of the first substrate that faces the plurality of RF supply terminals. A pair of RF output electrodes that is circuit-connected to the plurality of RF input electrodes and that is connected to the pair of connection pins may be formed on a second surface of the first substrate on a side opposite to the first surface.

In this case, the NFC chip may be disposed around the opening of the second surface, and is disposed at a location through which a virtual second straight line that vertically intersects the center of a virtual first straight line that connects the pair of RF output electrodes passes.

Furthermore, a DC power electrode, electrodes for data communication, and a ground electrode that are circuit-connected to the NFC chip may be arranged to form a group in one area around the opening in the first surface of the first substrate.

In this case, the DC power electrode, the electrodes for data communication, and the ground electrode may be disposed at locations at which the DC power electrode, the electrodes for data communication, and the ground electrode face the plurality of RF input electrodes with the opening interposed therebetween.

Furthermore, four RF input electrodes may be arranged in a row around the opening.

Furthermore, the body may include a first body detachably coupled to the front end of the handpiece and having the first substrate fixed and installed therein and a second body detachably coupled to the first body, having the second substrate movably installed therein, and having a plurality of holes through which the micro needles are able to enter and exit formed at a front end thereof.

Furthermore, the needle tip may further include a needle cap detachably coupled to a front end of the body in which the micro needles are disposed.

### [Advantageous Effects of Invention]

According to the needle tip structure for a handpiece according to an embodiment of the present disclosure, the NFC chip in which information related to a corresponding needle tip is stored is installed on the substrate within the needle tip. Accordingly, it is possible to provide usefulness in inventory management, history management, and shipment management of a needle tip because wireless communication with an external NFC terminal can be performed in a contactless manner and information stored in the NFC chip can be read/written.

In particular, there are advantages in that a manufacturer can use the needle tip to track and manage the production, distribution, and sales processes of the needle tip and the history and inventory management of a product is further facilitated because the movement and quality of the needle tip can be tracked by assigning a unique identifier to each needle tip. Furthermore, there are advantages in that a genuine product can be certified and illegal duplication can be prevented because a digital signature is included in the needle tip and the illegal distribution of the needle tip can also be prevented because information on a country in which the needle tip is sold is written in the needle tip and a device including the needle tip is driven only when a country in which the device is sold and a country in which the needle tip is sold are matched. Furthermore, in terms of the production of the needle tip, a production process can be simplified and production efficiency can be improved because various types of information can be written in the needle tip in an individually packaged state after all of production processes are finished.

Furthermore, in terms of practical use of the needle tip, the specifications of individually packaged needle tip products can be easily identified by reading them with an NFC terminal, thereby providing convenience and efficiency in use. Furthermore, it is possible to prevent a problem in that an error occurs in a procedure due to a needle tip not fit for the purpose of use, which is mounted on the handpiece, because an user can check accurate specification information of the needle tip even without opening packaging including the needle tip.

Furthermore, there are advantages in that a user can conveniently perform a procedure even without manually operating the switch included in the handpiece and efficiency and accuracy of a procedure can be improved because after the needle tip is mounted on the handpiece, the handpiece can automatically drive the driving unit and the RF generation unit in accordance with the type of needle tip mounted on the handpiece by accessing information stored in the NFC chip of the needle tip in a wired manner.

### [Brief Description of Drawings]

FIG. 1 is a perspective view illustrating a form in which a needle tip according to an embodiment of the present disclosure has been mounted on a handpiece.
FIG. 2 is a perspective view illustrating a form in which the needle tip according to an embodiment of the present disclosure has been separated from the handpiece.
FIG. 3 is a separation perspective view illustrating a construction of the needle tip in detail according to an embodiment of the present disclosure.
FIG. 4 is a cross-sectional view illustrating a cross-sectional structure of the needle tip according to an embodiment of the present disclosure.
FIG. 5 is a perspective view illustrating a form in which an NFC chip has been mounted on a first substrate of the needle tip.
FIG. 6 is a plan view of FIG. 5.
FIG. 7 is a rear view of FIG. 5.
FIG. 8 is a perspective view illustrating a structure of a front end part of the handpiece on which the needle tip according to an embodiment of the present disclosure is mounted.
FIG. 9 is a concept view conceptually illustrating a form in which data are transmitted between the needle tip on which the NFC chip is mounted and the handpiece.
FIG. 10 is a concept view illustrating a form in which information of the needle tip encased in packaging is accessed wirelessly through an external NFC terminal.

### [Description of Embodiments]

Hereinafter, embodiments of the present disclosure are described in detail with reference to the accompanying drawings.

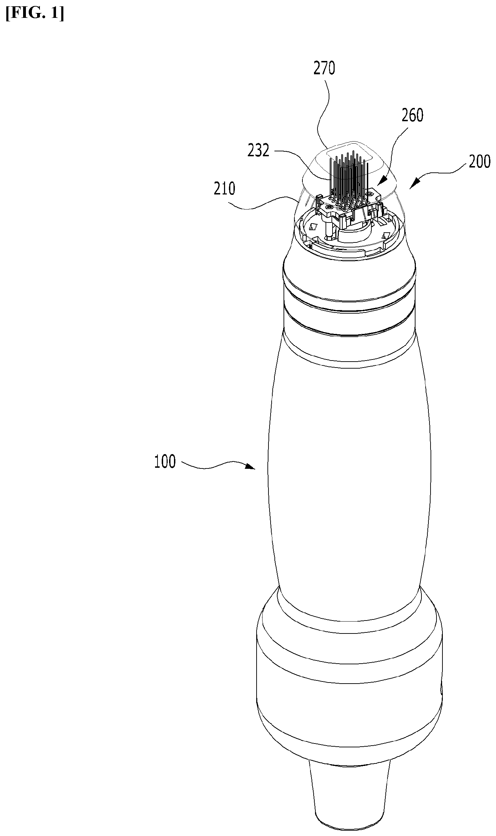

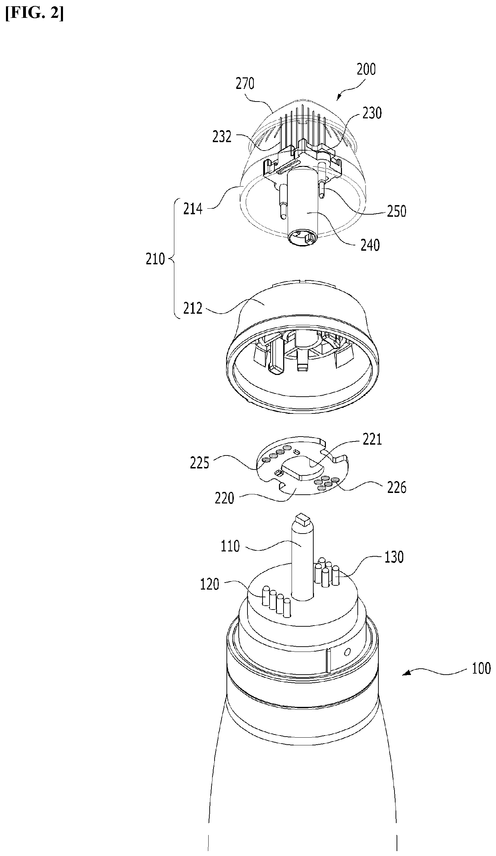

FIG. 1 illustrates a form in which a needle tip according to an embodiment of the present disclosure has been mounted on the front end of a handpiece. FIG. 2 illustrates a form in which the needle tip according to an embodiment of the present disclosure has been separated from the front end of the handpiece.

Referring to FIGS. 1 and 2, a needle tip 200 according to an embodiment of the present disclosure is mounted on the front end (i.e., the top of a handpiece 100 in FIG. 1) of the handpiece 100. The needle tip 200 is mounted on the front end of the handpiece 100 in a cartridge manner in a way to be replaceable, if necessary.

The needle tip 200 includes a plurality of micro needles 232 to which a radio frequency (RF) transmitted by the handpiece 100 is applied. The micro needle 232 included in the needle tip 200 penetrate a target skin tissue of a procedure target and transfers RF heat energy to the skin tissue.

The needle tip 200 may be provided in several types in which the number of micro needles 232 and an arrangement form of the micro needles 232 are different according to the purpose of use. An user (or practitioner) may select the needle tip 200 that is fit for a procedure, may mount the needle tip 200 on the handpiece 100, and may perform a procedure task.

The handpiece 100 is connected to a main body of a skin beauty treatment device (not illustrated) through a cable, and it drives a needle assembly 260 within the needle tip 200 and transfers an RF current to the needle assembly 260.

Although not illustrated in the drawings, the handpiece 100 includes a driving unit that drives the micro needle 232 within the needle tip 200 back and forth in a straight line, an RF generation unit that generates an RF that is transferred to the micro needle 232, and a control unit that controls the driving unit and the RF generation unit.

The driving unit may move the needle assembly 260 on which the micro needle 232 has been mounted in forward and backward directions (in up and down directions in FIG. 1) within a predetermined stroke range. The RF generation unit may transfer power supplied by the main body of the skin beauty treatment device to the micro needle 232 by converting the power in the form of an RF current pulse. Various forms, such as a motor, a ball screw, a pneumatic actuator, and an electromagnet actuator, may be applied as the driving unit.

The control unit is connected to various manipulation buttons provided on an external surface or internal surface of the handpiece 100, and may control operations of the driving unit and the RF generation unit by generating a control signal through a manipulation button by a user or a manipulation of a device interface.

As illustrated in FIG. 2, a part of a driving shaft 110 connected to the driving unit within the handpiece 100 is exposed to the outside of the front end of the handpiece 100. The driving shaft 110 that is exposed to the front end of the handpiece 100 is coupled to a holder 240 of the needle assembly 260 within the needle tip 200. Accordingly, when the driving unit operates, the needle assembly 260 coupled to the driving shaft 110 may be moved in forward and backward directions (i.e., in up and down directions in FIG. 2) of the handpiece 100.

A plurality of RF supply terminals 120 that supplies an RF current generated by the RF generation unit to the micro needle 232 within the needle tip 200 is provided around the driving shaft 110 exposed to the front end of the handpiece 100.

The plurality of RF supply terminals 120 disposed around the driving shaft 110 is electrically connected to a first substrate 220 of the needle tip 200 when the needle tip 200 is mounted on the front end of the handpiece 100. That is, the plurality of RF supply terminals 120 is electrically connected to a plurality of RF input electrodes 225 formed in the first substrate 220 of the needle tip 200, and transfers an RF current to the needle assembly 260 within the needle tip 200.

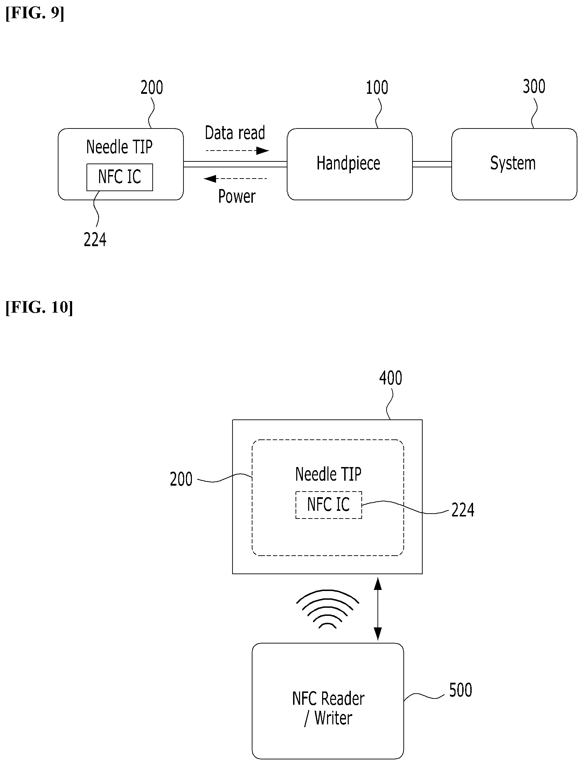

The needle tip 200 according to an embodiment of the present disclosure has a near field communication (NFC) function which enables short distance communication within an external terminal. That is, an NFC chip 224 is mounted within the needle tip 200 according to an embodiment of the present disclosure, and may perform short-distance wireless communication with an external NFC terminal 500 (refer to FIG. 10) in a contactless manner.

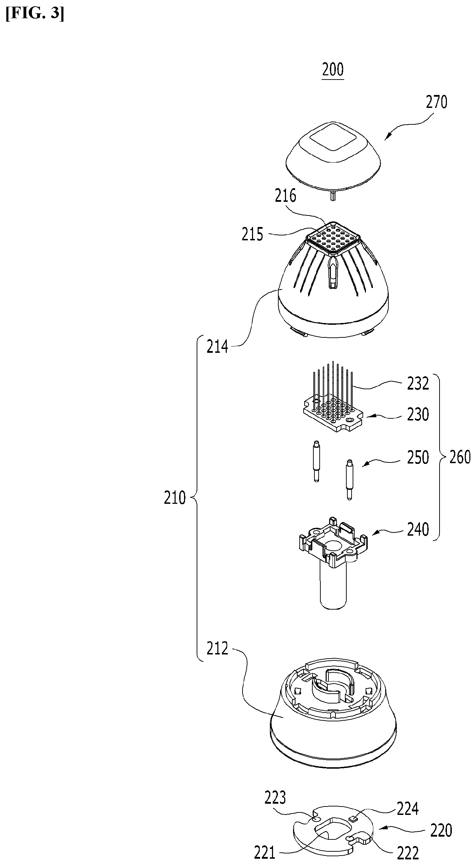

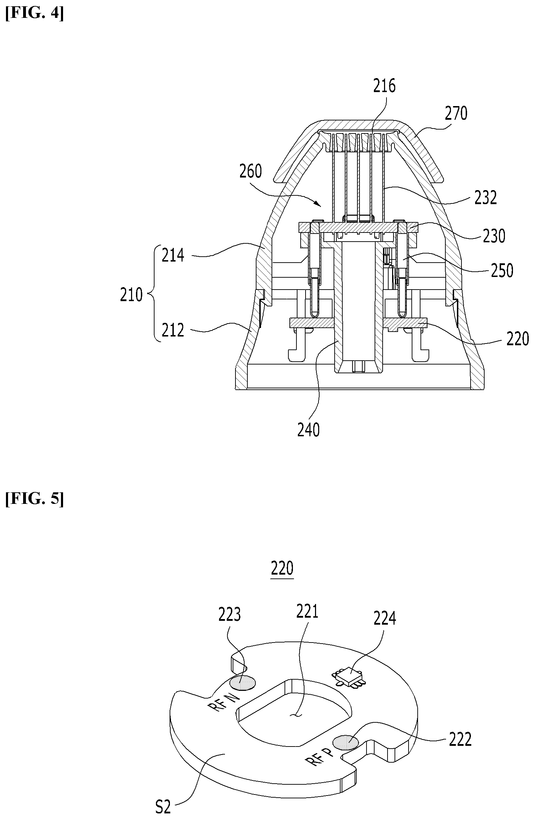

FIG. 3 is a separation perspective view illustrating components of the needle tip in which the NFC chip has been embedded in detail. FIG. 4 is a cross-sectional view illustrating a cross-sectional structure of the needle tip in which the NFC chip has been embedded.

Referring to FIGS. 3 and 4, the needle tip 200 includes a body 210, the needle assembly 260, and a needle cap 270.

The body 210 has an internal space in which the needle assembly 260 can be accommodated, and is detachably coupled to the front end of the handpiece 100. The driving shaft 110 of the handpiece 100 is connected to the internal space of the body 210. The needle assembly 260 capable of moving in the forward and backward directions (in the up and down directions in the drawings) of the handpiece 100 is disposed in the internal space.

The body 210 includes a first body 212 and a second body 214 having two spit shapes. That is, the body 210 is formed by the mutual coupling of the first body 212 and the second body 214 that are split into two. In this case, the first body 212 and the second body 214 may be detachably coupled. As the body 210 has a structure in which the body can be separated into the two bodies as described above, the needle assembly 260 can be easily installed in the internal space of the body 210.

The first body 212 that forms a lower (rear part) structure of the body 210 is detachably coupled to the front end of the handpiece 100. The first substrate 220 that electrically connects the handpiece 100 and the needle assembly 260 is installed within the first body 212. That is, the first substrate 220 is installed as a structure in which the first substrate 220 has been fixed within the first body 212, and functions to electrically connect the plurality of RF supply terminals 120 exposed to the front end of the handpiece 100 and the needle assembly 260.

The second body 214 that forms an upper (front part) structure of the body 210 is detachably coupled to a front end (top) of the first body 212. The needle assembly 260 that is connected to the driving shaft 110 of the handpiece 100 is disposed within the second body 214. The needle assembly 260 connected to the driving shaft 110 of the handpiece 100 may be moved in the forward and backward directions (in the up and down directions in the drawings) within the second body 214 when the driving shaft 110 operates.

A contact surface 215 that is closely attached to the skin of a procedure subject is formed at a front end of the second body 214. Furthermore, a plurality of needle holes 216 is formed in the contact surface 215 so that the plurality of micro needles 232 provided in the needle assembly 260 can enter the inside of the contact surface 215 and exit the contact surface 215.

Upon procedure using the needle tip 200, when the needle assembly 260 is advanced through the driving unit of the handpiece 100 in the state in which the skin of the procedure target has been pressed on the contact surface 215 of the second body 214, the micro needle 232 is drawn to the outside of the needle hole 216, and penetrates a skin tissue within the skin. A skin beauty treatment task and a treatment task can be performed by applying an RF to the micro needle 232 through the RF generation unit in this state so that heat energy is transferred to the skin tissue.

The first substrate 220 fixed within the first body 212 functions to electrically connect the handpiece 100 and the needle assembly 260. A first surface S1 of the first substrate 220, which is disposed at the bottom of the first substrate 220 in FIG. 3, is connected to the plurality of RF supply terminals 120 of the handpiece 100. A second surface S2 of the first substrate 220, which is disposed at the top of the first substrate 220 on a side opposite to the first surface S1, is connected to a pair of connection pins 250 of the needle assembly 260. In this case, the first surface S1 of the first substrate 220 refers to a surface in a direction toward the handpiece 100. The second surface S2 refers to a surface in a direction toward the micro needle 232.

The needle assembly 260 includes the plurality of micro needles 232, a second substrate 230 on which the plurality of micro needles 232 is mounted, the holder 240 that supports the second substrate 230, and the pair of connection pins 250 connected to the second substrate 230 through the holder 240. The needle assembly 260 having such a construction is connected to the driving shaft 110 of the handpiece 100, and may be moved in the forward and backward directions within a predetermined stroke range along with the driving shaft 110. Accordingly, the micro needle 232 may protrude to the outside of the second body 214 in the forward and backward directions.

The second substrate 230 on which the plurality of micro needles 232 is mounted is seated at the end of the holder 240 on one side thereof, and is coupled to the holder 240.

The holder 240 supports the second substrate 230 on which the plurality of micro needles 232 is mounted and also enables a connection with the driving shaft 110 of the handpiece 100. That is, a pillar unit of the holder 240 at the center thereof is coupled to the driving shaft 110 of the handpiece 100 through the top of the first body 212 and the first substrate 220.

The pair of connection pins 250 is connected to both opposite sides of the second substrate 230. The pair of connection pins 250 connected to the second substrate 230 is electrically connected to the first substrate 220 through the holder 240. In this case, the end of each of the pair of connection pins 250 may be connected to the first substrate 220 through a closed top of the first body 212. As the pair of connection pins 250 connected to the second substrate 230 as described above electrically connects the first substrate 220 and the second substrate 230, an RF current that is introduced from the handpiece 100 to the first substrate 220 can be introduced into the second substrate 230 through the pair of connection pins 250.

The pair of connection pins 250 that connects the first substrate 220 and the second substrate 230 may have a pogo pin structure having a retractile length. That is, each of the pair of the connection pins 250 is formed to have the pogo pin structure in which a spring is mounted within the connection pin 250, and may be constructed to be retractile in a length direction thereof. As the pair of connection pins 250 is each constructed to have the pogo pin structure in which each connection pin is retractile in the length direction as described above, the second substrate 230 can also maintain an electrical connection state with the first substrate 220 through the pair of connection pins 250 that is retractile although the needle assembly 260 is moved in the forward and backward directions within the second body 214.

Furthermore, the needle cap 270 capable of covering the micro needle 232 so that the micro needle 232 is not exposed to the outside is coupled to the front end of the second body 214. In this case, the needle cap 270 is coupled to the front end of the second body 214 as a structure in which the needle cap 270 is detachable from the front end of the second body 214. Accordingly, a procedure task can be performed in the state in which the needle hole 216 has been exposed to the outside by separating the needle cap 270 from the second body 214.

The needle tip 200 according to an embodiment of the present disclosure includes the NFC chip 224 that enables short-distance wireless communication with the external NFC terminal 500 (refer to FIG. 10). The NFC chip 224 of the needle tip 200 is installed in a part of the first substrate 220 that electrically connects the needle assembly 260 and the handpiece 100.

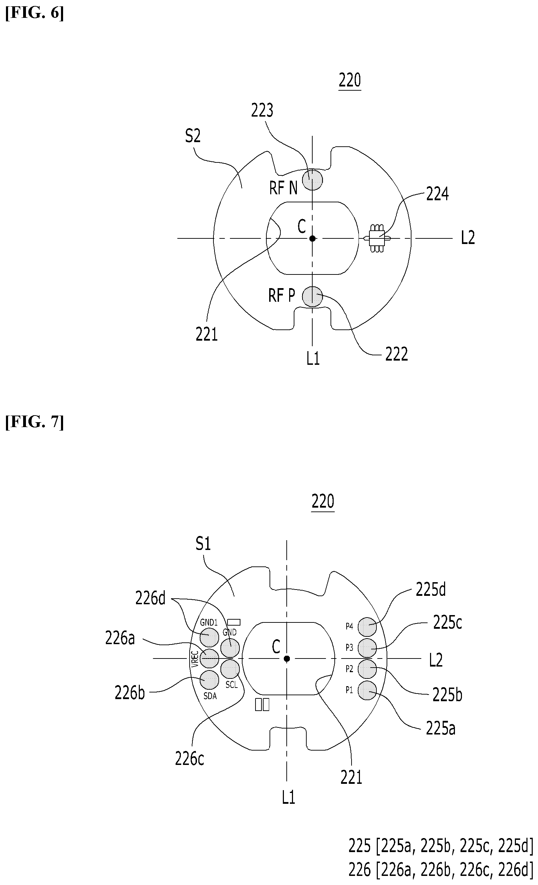

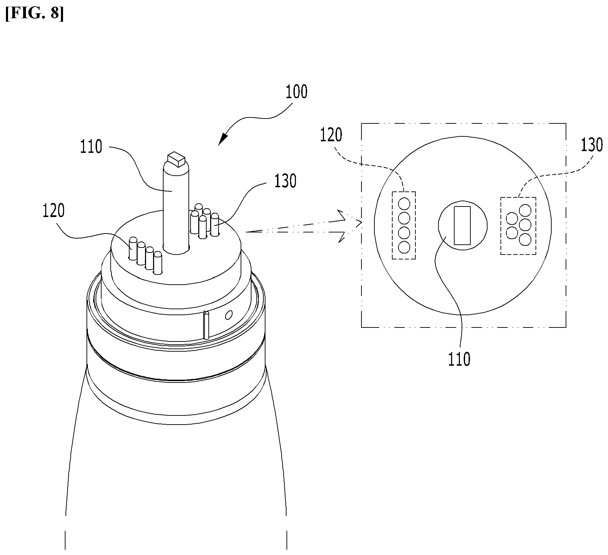

FIG. 5 is a perspective view illustrating a form in which the NFC chip has been installed on the first substrate of the needle tip. FIGS. 6 and 7 illustrate plan and rear structures of the first substrate illustrated in FIG. 5. Furthermore, FIG. 8 illustrates a structure of the front end of the handpiece in which the plurality of RF supply terminals and signal transmission terminals connected to the plurality of RF input electrodes and signal transmission electrodes formed in the first substrate, respectively, are arranged.

Referring to FIGS. 5 to 8, in the needle tip 200 according to an embodiment of the present disclosure, the first substrate 220 is fixed and installed in the internal space of the first body 212 that is directly coupled to the handpiece 100.

An opening 221 through which the pillar unit of the holder 240 penetrates is formed at the center of the first substrate 220. Furthermore, the plurality of RF supply terminals 120 of the handpiece 100 is connected to one surface of the first substrate 220, and the pair of connection pins 250 of the needle assembly 260 is connected to the other surface of the first substrate 220.

That is, the plurality of RF input electrodes 225 connected to the plurality of RF supply terminals 120 of the handpiece 100, respectively, is formed on the first surface S1 of the first substrate 220 toward the handpiece 100. A pair of RF output electrodes 222 and 223 that is electrically connected to the plurality of RF input electrodes 225 and to which the pair of connection pins 250 of the needle assembly 260 is connected, respectively, is formed on the second surface S2 on a side opposite to the first surface S1. In this case, the pair of RF output electrodes 222 and 223 formed on the second surface S2 of the first substrate 220 is disposed at positions that are symmetrical to each other with the opening 221 at the center of the first substrate 220 interposed therebetween.

According to an embodiment, the NFC chip 224 may be installed in a part of the first substrate 220 in which a plurality of electrode patterns that electrically connects the pair of connection pins 250 connected to the second substrate 230 and the plurality of RF supply terminals 120 of the handpiece 100 is disposed. The NFC chip 224 installed in the first substrate 220 stores various types of data information related to the specifications of the needle tip 200, and may perform contactless wireless communication with the external NFC terminal 500.

Specifically, the NFC chip 224 may be installed in the area of the second surface S2 of the first substrate 220 toward the needle assembly 260. That is, the NFC chip 224 may be installed to be disposed in a surrounding area of the opening 221, which is relatively distant from the pair of RF output electrodes 222 and 223 in the area of the second surface S2 of the first substrate 220.

In this case, the NFC chip 224 disposed in the surrounding area of the opening 221 of the second surface S2 may be disposed at a location around the opening 221 through which a virtual second straight line L2 that vertically intersects the center C of a virtual first straight line L1 that connects the pair of RF output electrodes 222 and 223 passes (refer to FIG. 6).

If the NFC chip 224 is disposed to have such a structure, intervals between the NFC chip 224 and the pair of RF output electrodes 222 and 223 on both sides of the NFC chip 224 can be identically maintained, and the NFC chip 224 can be disposed at a location as far away as possible from the pair of RF output electrodes 222 and 223 on both sides of the NFC chip 224. Accordingly, a reduction of NFC communication performance, which occurs due to electromagnetic interference between the NFC chip 224 and the pair of RF output electrodes 222 and 223 on both sides of the NFC chip 224 can be minimized, and reliability of communication performance can be improved.

An electrode group 226 for signal transmission that is connected to the NFC chip 224 mounted on the second surface S2 in the form of a circuit pattern, that is, a DC power electrode 226a, electrodes 226b and 226c for data communication, and a ground electrode 226d, are arranged to form a group in one area around the opening 221 on the first surface S1 of the first substrate 220.

In accordance with the electrode group 226, a plurality of terminals 130 for signal transmission, which is connected to the DC power electrode 226a, electrodes 226b and 226c for data communication, and ground electrode 226d of the first substrate 220, is arranged to form a group in the same array form around the driving shaft 110 of the handpiece 100.

The DC power electrode 226a formed in the first substrate 220 functions as a power line that supplies power to the NFC chip 224. Accordingly, the NFC chip 224 may perform short distance communication with the external NFC terminal 500 by being supplied with power from the handpiece 100 through the DC power electrode 226a.

Furthermore, the electrodes 226b and 226c for data communication function as signal lines for communication with the handpiece 100. The electrodes 226b and 226c for data communication include serial data (SDA) 226b and a serial clock (SCL) 226c. The SDA 226b and the SCL 226c are directly connected to the control unit (main controller) of the handpiece 100, and may exchange data with the control unit.

In this case, the SDA 226b functions as a signal line for the transmission and reception of data. The NFC chip 224 may transmit and receive data to and from the control unit of the handpiece 100 through the SDA 226b. Furthermore, the SCL 226c functions as a signal line that synchronizes a data transfer rate, and provides timing while data are transmitted by transmitting a signal at a predetermined cycle when the data are transmitted. Furthermore, the ground electrode 226d stabilizes a change in the voltage or current, maintains the stability of a circuit by removing noise generated within the circuit, and improves performance.

The DC power electrode 226a, the electrodes 226b and 226c for data communication, and the ground electrode 226d form the electrode group 226 for signal transmission in which the DC power electrode 226a, the electrodes 226b and 226c for data communication, and the ground electrode 226d are arranged to form a group on the first surface S1 of the first substrate 220 disposed on the side opposite to the NFC chip 224.

Furthermore, as described above, the plurality of RF input electrode 225 connected to the plurality of RF supply terminals 120 exposed to the front end of the handpiece 100, respectively, is formed on the first surface S1 of the first substrate 220. In this case, four RF input electrode 225 may be arranged and formed in a row in parallel to the virtual first straight line L1 in the surrounding area of the opening 221.

In this case, first and second RF input electrodes 225a and 225b, among the four RF input electrodes 225a to 225d, may be connected to the first RF output electrode 222 disposed on the second surface S2. Third and fourth RF input electrodes 225c and 225d, among the four RF input electrodes 225a to 225d, may be connected to the second RF output electrode 223 disposed on the second surface S2. In this case, the first RF output electrode 222 is an electrode from which an RF signal (+) is output, and the second RF output electrode 223 is an electrode from which an RF signal (-) is output.

The plurality of RF input electrodes 225 may be disposed at locations at which the plurality of RF input electrodes 225 faces the DC power electrode 226a, the electrodes 226b and 226c for data communication, and the ground electrode 226d that are arranged to form a group with the opening 221 at the center C of the opening 221 interposed therebetween (refer to FIG. 7).

That is, the electrode group, including the DC power electrode 226a, the electrodes 226b and 226c for data communication, and the ground electrode 226d, and the four RF input electrodes 225a to 225d may be disposed at the locations at which the electrode group and the four RF input electrodes 225a to 225d face each other in a direction toward the virtual second straight line L2 that passes through the center C of the opening 221.

When the electrode group and the four RF input electrodes 225a to 225d are disposed as described above, the plurality of RF input electrodes 225a to 225d through which an RF current flows and the four electrodes 226a to 226d for signal transmission that form a group in the first substrate 220 are disposed at the locations as far away as possible. Accordingly, a reduction of communication performance attributable to electromagnetic interference between the electrodes disposed on both sides can be prevented, and reliability of communication performance can be improved.

In contrast, if the NFC chip 224 is constructed to operate by wirelessly receiving power from the external NFC terminal 500 without receiving power from the handpiece 100, power consumption of the NFC chip 224 may be increased due to electromagnetic interference between the electrodes on both sides. Accordingly, a power efficiency reduction problem of the NFC chip 224, which occurs because the NFC chip 224 consumes additional power due to electromagnetic interference between the electrodes on both sides through such a construction, can be prevented. The coverage reduction and data transmission instability of the NFC chip, which occur because an NFC signal is attenuated due to electromagnetic interference, can also be overcome.

FIG. 9 conceptually illustrates a form in which power is supplied and data are transmitted between the needle tip on which the NFC chip is mounted and the handpiece.

As illustrated in FIG. 9, the handpiece 100 connected to a skin beauty treatment device(or system) 300 may supply power for an operation of the NFC chip 224 mounted within the needle tip 200, and may drive the driving unit and the RF generation unit through the control unit of the handpiece 100 by reading data information stored in the NFC chip 224.

FIG. 10 illustrates a form in which the needle tip encased in packaging is wirelessly accessed through an external NFC terminal.

As illustrated in FIG. 10, when the needle tip 200 is encased in the packaging 400 in the form of a product, a user (e.g., a practitioner) can easily check product specifications of the needle tip 200 by reading product information stored in the NFC chip 224 of the needle tip 200 within packaging 400 through the external NFC terminal 500 (e.g., an NFC reader) in the state in which the packaging 400 has been packaged without being directly opened. Furthermore, data information may be written and stored in the NFC chip 224 of the needle tip 200 within the packaging 400 by using the external NFC terminal 500 (e.g., an NFC writer). As described above, data can be read from and written in the NFC chip 224 of the needle tip 200 by using the external NFC terminal 500. In this case, the external NFC terminal 500 may include an application (App) that is installed in a mobile phone.

As described above, according to an embodiment of the present disclosure, wireless communication with the external NFC terminal 500 can be performed in a contactless manner and information stored in the NFC chip 224 can be read and written because the NFC chip 224 in which related data information of a corresponding needle tip is stored is installed in the first substrate 220 of the needle tip 200. Accordingly, the inventory management, history management, and shipment management of the needle tip 200 can be easily performed.

In particular, there are advantages in that a manufacturer of the needle tip 200 can use the needle tip to track and manage the production, distribution, and sales processes of the needle tip and the history and inventory management of a product is further facilitated because the movement and quality of the needle tip can be tracked by assigning a unique identifier to each needle tip. Furthermore, there are advantages in that a genuine product can be certified and illegal duplication can be prevented because a digital signature is included in the needle tip 200.

Furthermore, use convenience and efficiency of the needle tip 200 can be improved because the specifications of the needle tip 200 can be checked easily and conveniently by reading the specifications of the needle tip 200 in an individually packaged state through the external NFC terminal 500. Furthermore, it is possible to prevent a problem in that an user (or a practitioner) commits an error in a procedure due to a needle tip not fit for the purpose of use, which is mounted on the handpiece, because the user can accurately check the specifications of the needle tip 200 and whether a product is a genuine product even in the state in which the user has not directly opened the packaging of the needle tip 200.

Furthermore, when the needle tip 200 is mounted on the handpiece 100 for a procedure task, the control unit of the handpiece 100 can access information stored in the NFC chip 224 of the needle tip 200 in a wired way and automatically drive the driving unit and the RF generation unit by transmitting a control signal suitable for the specifications of the needle tip 200. Accordingly, an user (or practitioner) can conveniently perform a procedure even without manually operating a manipulation button provided in the handpiece 100, and thus efficiency and accuracy of the procedure can be improved.

Furthermore, an additional instrument or wire for the installation of the NFC chip 224 is not required because the NFC chip 224 is installed in a part of the first substrate 220 in which various electrode patterns that connect the handpiece 100 and the pair of connection pins 250 are disposed. A device structure within the needle tip 200 does not become complex and costs according to the installation of the NFC chip 224 can also be reduced because the NFC chip 224 can be simply installed by changing only a circuit pattern structure of the first substrate 220.

In particular, the NFC chip 224 is installed around the opening 221 on the second surface S2 of the first substrate 220, but is installed at the location through which the virtual second straight line L2 that vertically intersects the center C of the virtual first straight line L1 that connects the pair of RF output electrodes 222 and 223 on both sides of the center C passes. The distances between the NFC chip 224 and the pair of RF output electrodes 222 and 223 on both sides of the center C are spaced apart from each other as much as possible. Accordingly, a communication error problem that occurs due to electromagnetic interference between the NFC chip 224 and the pair of RF output electrodes 222 and 223 can be prevented, and NFC communication performance and reliability can be improved.

Furthermore, the DC power electrode 226a, the electrodes 226b and 226c for data communication, and the ground electrode 226d that are arranged to form a group in a way to be circuit-connected to the NFC chip 224 on the first surface S1 of the first substrate 220 are disposed at the locations at which the DC power electrode 226a, the electrodes 226b and 226c for data communication, and the ground electrode 226d are symmetrical to the plurality of RF input electrodes 225 with the opening 221 interposed therebetween. Accordingly, a reduction of communication performance attributable to electromagnetic interference can be prevented and reliability of NFC communication performance can be improved because a separation distance between the plurality of RF input electrodes 225 and the electrodes 226a to 226d for signal transmission through which an RF current flows can be maximized.

Furthermore, if the NFC chip 224 is constructed to operate by directly receiving power from the external NFC terminal 500 wirelessly, a power efficiency reduction problem of the NFC chip 224, which occurs because the NFC chip 224 consumes additional power due to electromagnetic interference between the electrodes on both sides of the NFC chip 224, can be overcome. Coverage reduction and data transmission instability problems of the NFC chip 224, which occur because an NFC signal is attenuated due to electromagnetic interference, can also be overcome.

Furthermore, the body 210 of the needle tip 200 is constructed to be separated into the first body 212 and the second body 214 which are mutually detachable. Accordingly, the needle assembly 260 can be further easily installed within the second body 214 because the second body 214 is separated from the first body 212. If NFC communication is not smooth, after the second body 214 is separated from the first body 212, the external NFC terminal may be made closest to the NFC chip 224 installed in the first body 212 for smooth NFC communication.

Although the embodiments of the present disclosure have been described above, the scope of the present disclosure is not limited to only such specific embodiments, and a person having ordinary knowledge in a corresponding field may properly change the present disclosure without departing from the category of the claims of the present disclosure.

### [Description of reference numerals]

| | | | |
|---|---|---|---|
| 100: | handpiece | 110: | driving shaft |
| 120: | RF supply terminal | | |
| 130: | terminal for signal transmission | | |
| 200: | needle tip | 210: | body |
| 212: | first body | 214: | second body |
| 216: | needle hole | 220: | first substrate |
| 221: | opening | 222, 223: | RF output electrode |
| 224: | NFC chip | 225: | RF input electrode |
| 226a: | DC power electrode | | |
| 226b, 226c: | electrode for data communication | | |
| 226d: | ground electrode | 230: | second substrate |
| 232: | needle | 240: | holder |
| 250: | connection pin | 260: | needle assembly |
| 270: | needle cap | 400: | packaging |
| 500: | NFC terminal | | |

## Claims

1. A needle tip for a handpiece having a near field communication (NFC) function and provided at a front end of the handpiece for skin beauty treatment and treatment in a way to be replaceable and mountable, the needle tip comprising:
a body detachably coupled to the front end of the handpiece;
a first substrate fixed and installed within the body, having an opening formed at a center thereof, and electrically connected to a plurality of radio frequency (RF) supply terminals exposed to the front end of the handpiece;
a second substrate movably installed within the body and having a plurality of micro needles mounted thereon;
a holder having one end coupled to a driving shaft of the handpiece through the opening of the first substrate and having the other end coupled to the second substrate; and
a pair of connection pins connected to the second substrate and configured to electrically connect the first substrate and the second substrate,
wherein an NFC chip in which information related to the needle tip is stored and configured to perform contactless wireless communication with an external NFC terminal is installed in the first substrate.

2. The needle tip of claim 1, wherein:
a plurality of RF input electrodes connected to the plurality of RF supply terminals is formed on a first surface of the first substrate that faces the plurality of RF supply terminals, and
a pair of RF output electrodes that is circuit-connected to the plurality of RF input electrodes and that is connected to the pair of connection pins is formed on a second surface of the first substrate on a side opposite to the first surface.

3. The needle tip of claim 2, wherein the NFC chip is disposed around the opening of the second surface, and is disposed at a location through which a virtual second straight line that vertically intersects a center of a virtual first straight line that connects the pair of RF output electrodes passes.

4. The needle tip of claim 2, wherein a DC power electrode, electrodes for data communication, and a ground electrode that are circuit-connected to the NFC chip are arranged to form a group in one area around the opening in the first surface of the first substrate.

5. The needle tip of claim 4, wherein the DC power electrode, the electrodes for data communication, and the ground electrode are disposed at locations at which the DC power electrode, the electrodes for data communication, and the ground electrode face the plurality of RF input electrodes with the opening interposed therebetween.

6. The needle tip of claim 2, wherein four RF input electrodes are arranged in a row around the opening.

7. The needle tip of claim 1, wherein the body comprises:
a first body detachably coupled to the front end of the handpiece and having the first substrate fixed and installed therein; and
a second body detachably coupled to the first body, having the second substrate movably installed therein, and having a plurality of holes through which the micro needles are able to enter and exit formed at a front end thereof.

8. The needle tip of claim 1, further comprising a needle cap detachably coupled to a front end of the body in which the micro needles are disposed.
